# EUROPÄISCHE PATENTANMELDUNG

(11) **EP 2 407 187 A1**
(43) Veröffentlichungstag der Anmeldung: **18.01.2012**
(21) Anmeldenummer: 10075304.5
(22) Anmeldetag: 15.07.2010
(51) Int. Cl.: A61M 1/10, A61M 1/12

(54) **Blutpumpe für die invasive Anwendung innerhalb eines Körpers eines Patienten**

(71) Anmelder: ECP Entwicklungsgesellschaft mbH, 12247 Berlin (DE)
(72) Erfinder: Töllner, Thomas, 12047 Berlin (DE); Scheckel, Mario, 13187 Berlin (DE)
(74) Vertreter: Golkowsky, Stefan

(57) **Zusammenfassung**

Die Erfindung bezieht sich auf eine Blutpumpe für die invasive Anwendung innerhalb eines Körpers eines Patienten mit einem um eine Rotationsachse antreibbaren radial komprimierbaren und expandierbaren Rotor (43, 27), der eine Nabe (18, 28) und wenigstens ein daran befestigtes Schaufelblatt (14, 15, 16, 17, 29, 30) aufweist, sowie mit einem Gehäuse (19, 19'), das durch eine Axialdehnung oder Axialkompression in radialer Richtung komprimierbar oder expandierbar ist. Die Aufgabe, auf möglichst einfache Weise sowohl den Rotor als auch das Gehäuse expandierbar und komprimierbar zu machen, wird gemäß der Erfindung dadurch gelöst, dass ein die Nabe (18) in Längsrichtung durchsetzender Steuerkörper (21) vorgesehen ist, der auf der distalen Seite des Rotors mit dem Gehäuse derart gekoppelt ist, dass er durch eine Bewegung in Längsrichtung gegenüber dem Gehäuse auf dieses Zug-und/oder Druckkräfte ausübt.

## Beschreibung

Die Erfindung liegt auf dem Gebiet des Maschinenbaus, insbesondere der Mikromechanik, und befasst sich speziell mit Blutpumpen.

Der Einsatz der Erfindung liegt insbesondere auf medizinischem Gebiet, wo entsprechende Blutpumpen in verschiedenen Ausprägungen zu verschiedenen Zwecken bereits bekannt geworden sind. Solche Pumpen können invasiv angewendet und dazu über ein Blutgefäß in den Körper eines Patienten eingebracht und dort betrieben werden. Besonders vorteilhaft sind derartige Pumpen beispielsweise in einer Herzkammer, speziell in einem linken Ventrikel, zur Herzunterstützung einsetzbar. Eine solche Pumpe wird zu diesem Zweck über eine Femoralarterie mittels eines Hohlkatheters eingeschoben und bis in das linke Ventrikel eines Patientenherzens eingeführt. Dort saugt die Blutpumpe Blut an und stößt dies in der Aorta wieder aus. Auf diese Weise kann die Herzfunktion wenigstens teilweise ersetzt oder unterstützt werden, um derart beispielsweise bei Eingriffen das Herz zu entlasten oder die Bedingungen für eine Erholung des Herzens des Patienten zu verbessern.

Eine derartige Pumpe weist einen Antrieb auf, der beispielsweise außerhalb des Patientenkörpers liegen kann und von dem aus die Antriebsbewegung mittels einer drehbaren Welle durch den Hohlkatheter zu einem Rotor übertragen wird. Es ist allerdings auch der Betrieb von Mikroturbinen in der Nähe der Blutpumpe für deren Antrieb bekannt.

Ein Problem bei der Effizienz derartiger Pumpen stellen die geringen Abmessungen dar, die für den Transport innerhalb des Patientenkörpers vorteilhaft sind. Eine derartige Pumpe bzw. der Rotor ist üblicherweise für den Transport radial komprimierbar, um danach für den eigentlichen Betrieb expandiert zu werden.

Es sind verschiedene Konstruktionen bekannt geworden, die eine Überführung des Rotors zwischen einem komprimierten und einem expandierten Zustand erlauben. Beispielsweise sind Konstruktionen mit Stützstreben aus Gedächtnislegierungen bekannt, die ihre Gestalt in Abhängigkeit von der Umgebungstemperatur ändern. Die Stützstrukturen können dann mit einer Membran bespannt sein, um die Schaufelblätter eines Rotors zu bilden.

Es sind auch einteilige Rotoren mit Naben bekannt, an denen einstückig Schaufelblätter befestigt sind, die aufgrund ihrer abgewogenen elastischen Eigenschaften an die Nabe anklappbar und von dieser abspreizbar sind. Oft geschieht die Kompression der Schaufelblätter durch das den Rotor umgebende Gehäuse, das zur Expansion aufgeweitet werden kann. Das Aufrichten der Schaufelblätter kann beispielsweise durch die Rotation des Rotors im Betrieb geschehen, indem die Schaufelblätter sich durch den entstehenden Fluidgegendruck bei der Rotation aufrichten.

Ein Problem bei der Konstruktion derartiger Blutpumpen besteht beispielsweise darin, dass die Anforderungen an die Materialeigenschaften des Werkstoffs, aus dem der Rotor oder speziell die Schaufelblätter bestehen, sehr hoch sind. Zum vollständigen Anklappen an die Nabe müssen die Schaufelblätter in lokal eingegrenzten Bereichen sehr stark verformt werden, und es ist auch wünschenswert, dass die für die Kompression notwendigen Kompressionskräfte gering bleiben, um den Rotor ohne großen Aufwand und ohne übermäßigen Verschleiß komprimieren zu können. Zu diesem Zweck sind bereits Kompositwerkstoffe zur Herstellung der Schaufelblätter vorgeschlagen worden.

Diese sehen einerseits einen Schaufelblattkörper aus einem relativ elastischen Material vor, andererseits zusätzliche Streben, die in einen Schaufelblattkörper integriert oder auf diesen aufgesetzt sein können und die das Schaufelblatt zumindest im expandierten Zustand stabilisieren und stützen.

Für eine sinnvolle Expansion sowohl des Rotors als auch des diesen umgebenden Gehäuses sind bereits verschiedene Vorschläge bekannt, wie beispielsweise derjenige, das Gehäuse durch die Expansion des Rotors mit zu expandieren oder das Gehäuse unabhängig von dem Rotor zu expandieren und dadurch die in dem Rotor ruhenden elastischen Kräfte wirken zu lassen, die die Schaufelblätter selbsttätig zumindest teilweise expandieren.

Entsprechende Konstruktionen sind beispielsweise aus der WO 03/103745 und der WO 94/05347 bekannt geworden. Dort wird beispielsweise auch vorgeschlagen, Längskräfte auf das Gehäuse aufzubringen, um dieses beispielsweise im Rahmen einer Zugbewegung axial zu stauchen und damit radial aufzuweiten.

Der vorliegenden Erfindung liegt die Aufgabe zugrunde, vor dem Hintergrund des Standes der Technik eine Blutpumpe der eingangs genannten Art so auszubilden, dass mit möglichst geringem konstruktivem Aufwand eine gute, möglichst kraftfreie Kompression eines Rotors und/oder des Gehäuses einer Blutpumpe ermöglicht wird, wobei im expandierten Zustand der Rotor möglichst in der gewünschten geometrischen Form stabilisiert ist.

Die Aufgabe wird mit den Merkmalen der unabhängigen Patentansprüche gelöst.

Dementsprechend ist zur Realisierung der Erfindung eine Blutpumpe mit einem Rotor vorgesehen, der um seine Rotationsachse antreibbar und radial komprimierbar und expandierbar ist, um im komprimierten Zustand durch ein Blutgefäß transportiert zu werden.

Zudem weist der Rotor eine Nabe und wenigstens ein an dieser befestigtes Schaufelblatt sowie ein Gehäuse auf, das durch eine Axialdehnung oder Axialkompression in radialer Richtung komprimierbar oder expandierbar ist.

Es ist zudem ein die Nabe in Längsrichtung durchsetzender Steuerkörper vorgesehen, der auf der distalen Seite des Rotors mit dem Gehäuse derart gekoppelt ist, dass er durch eine Bewegung in Längsrichtung gegenüber dem Gehäuse auf dieses Zug- und/oder Druckkräfte ausübt.

Obwohl es grundsätzlich bereits bekannt ist, entsprechende Pumpengehäuse durch Ausübung von Axialkräften zu komprimieren bzw. zu expandieren, ist bei der vorliegenden Erfindung hierzu keine Bewegung oder Kraftübertragung durch den Rotor bzw. die Nabe selbst erforderlich. Es kann vielmehr eine Betätigung durch einen Steuerkörper erfolgen, der die Nabe durchsetzt. Dieser kann entweder mit dem Rotor rotieren oder gegenüber diesem stillstehen. Es kann sich beispielsweise bei dem Steuerkörper um einen Führungsdraht handeln, der vor der Pumpe in den Körper eines Patienten eingeführt wird und über den die Pumpe zum Einsatzort geschoben werden kann.

Der Steuerkörper kann mit dem Gehäuse, insbesondere dessen distalem Ende, an der Fügestelle axial unverschiebbar verbunden sein. Dabei kann der Steuerkörper in der Gehäusewand drehbar gelagert sein.

Es kann vorteilhaft vorgesehen sein, dass der Steuerkörper auf der distalen Seite der Gehäusewand einen Anschlagkörper aufweist, der bei einer Zugbewegung in proximaler Richtung eine axiale Kompressionskraft auf die Gehäusewand ausübt und damit das Gehäuse radial expandiert.

Es kann zusätzlich oder alternativ dazu auch vorgesehen sein, dass der Steuerkörper auf der proximalen Seite der Gehäusewand einen Anschlagkörper aufweist, der bei einer Schubbewegung in distaler Richtung eine axiale Expansionskraft auf die Gehäusewand ausübt.

Die entsprechenden Anschlagkörper sollten lediglich größer sein als die Bohrung in der Gehäusewand, die der Steuerkörper durchsetzt. Die Anschlagkörper können auch derart an dem Führungskörper befestigt sein, dass sie bei Anwendung einer bestimmten Mindestkraft längs auf dem Steuerkörper verschiebbar sind. Dadurch wird eine zu starke Kompression oder Expansion des Gehäuses verhindert.

Es kann auch vorgesehen sein, dass die Anschlagkörper bei Überschreiten einer bestimmten Kraftschwelle durch die Bohrung in der Wand des Pumpengehäuses hindurchgepresst werden können, beispielsweise wenn der Führungskörper endgültig entfernt werden soll. Hierzu kann beispielsweise vorgesehen sein, dass die Anschlagkörper aus einem kompressiblen Material wie beispielsweise Schaumstoff bestehen.

Es kann auch vorgesehen sein, dass der distal oder proximal der Gehäusewand angeordnete Anschlagkörper auflösbar oder verformbar ist, derart, dass der Steuerkörper entfernbar ist. Auch diese Ausgestaltung kann das endgültige Entfernen des Führungskörpers trotz der Anschlagkörper erlauben.

Das Gehäuse der Pumpe kann vorteilhaft ein bewegliches Gittergeflecht aufweisen, um durch eine Kompression oder Expansion in Längsrichtung gleichzeitig in Radialrichtung eine Expansion oder Kompression zu erzeugen.

Das Gitter kann beispielsweise eine Struktur von Gitterstäben aufweisen, die wenigstens teilweise schraubenförmig um die Rotationsachse herum angeordnet sind.

Zur Abdichtung des Gehäuses kann dieses eine durch das Gittergeflecht gestützte Membran aufweisen. Die Membran kann mit dem Gittergeflecht vergossen sein. Das Gittergeflecht kann auch von einem Spritzgussmaterial umgossen sein.

Eine weitere Ausgestaltung der Erfindung bezieht sich gemäß Patentanspruch 11 auf eine Blutpumpe für die invasive Anwendung innerhalb des Körpers eines Patienten mit einem Gehäuse sowie einem im Betrieb in diesem angeordneten antreibbaren Rotor mit wenigstens einem Schaufelblatt, wobei der Rotor und/oder das Gehäuse radial komprimierbar und expandierbar ist, dadurch gekennzeichnet, dass der Rotor und/oder das Gehäuse wenigstens teilweise aus einem Werkstoff besteht, der in ausgewählten Bereichen gegenüber anderen Bereichen derart modifiziert ist, dass er sich in den ausgewählten Bereichen bezüglich mechanischer Materialeigenschaften von den anderen Bereichen unterscheidet.

Eine vorteilhafte Ausgestaltung der Erfindung sieht vor, dass die ausgewählten Bereiche sich von den anderen Bereichen durch eine physikalische und/oder chemische Struktur unterscheiden. Dabei können die ausgewählten Bereiche beispielsweise bezüglich ihrer kristallinen Struktur, Modifikation, Dichte, des Aufschäumgrades oder der chemischen Vernetzung oder anderer denkbarer physikalischer oder chemischer Ordnungskräfte auf molekularer oder atomarer Ebene von den anderen Bereichen verschieden sein; die Bereiche bestehen dabei jedoch aus demselben Grundstoff.

Es kann außerdem vorgesehen sein, dass die ausgewählten Bereiche während des Formverfahrens modifiziert sind oder dass die ausgewählten Bereiche nach dem Formverfahren modifiziert sind, oder auch, dass die Bereiche nach einem ersten Formprozess durch einen zusätzlichen Umformprozess modifiziert sind.

Besonders vorteilhaft kann dabei sein, wenn wenigstens ein Teil des Rotors, insbesondere eines Schaufelblattes, und/oder wenigstens ein Teil des Gehäuses einen durch Strahlung erweichbaren oder erhärtbaren Werkstoff aufweist und wenn durch selektive Strahlungshärtung ausgewählte Bereiche versteift oder durch selektive Strahlungserweichung entsprechend ausgewählte Bereiche erweicht sind. Dabei ist grundsätzlich denkbar, dass der Werkstoff in den ausgewählten Bereichen durch Strahlung, insbesondere Alpha-, Beta- oder Gammastrahlung und/oder Wärmestrahlung, modifizierbar ist. Mittels fokussierbarer Strahlung können auf diese Weise mechanische Stützstrukturen in Form von ausgewählten Bereichen in den Werkstoff des Rotors und/oder des Gehäuses eingeschrieben werden.

Bereits bei der Formgebung können die ausgewählten Bereiche durch spezielle Guss-, Spritzguss- oder Extrusionsverfahren erzeugt werden. Es sind auch Auftrag- oder Tauchverfahren denkbar, bei denen Schicht um Schicht aufwächst und dabei beispielsweise Stoffkonzentrationen, Lösungsmittelkonzentrationen oder Zusammensetzungsverhältnisse verändert werden. Auch ist es möglich, diese Strukturen durch tropfenweises Auftragen des Werkstoffes zu erzeugen, wobei die Zusammensetzung tropfenweise variiert werden kann.

Das oder die Schaufelblätter bestehen im Wesentlichen aus einem verformbaren, insbesondere elastischen Material, das zur Kompression des Rotors leicht verformbar ist. An dieses werden jedoch in der gestreckten Stellung beim Rotations-/Pumpbetrieb hohe Anforderungen an die mechanische Stabilität gestellt. Diese Anforderungen können durch eine Kombination verschiedener Werkstoffe wie einem nachgiebigen, flexiblen Werkstoff und einer Strebe aus einem härteren Werkstoff als Kompositbauteil erfüllt werden.

Eine besonders einfache Ausgestaltung eines solchen Kompositbauteils wird durch die Erfindung geliefert, indem der Schaufelblattkörper einen strahlungshärtbaren oder -erweichbaren Werkstoff aufweist und ausgewählte Bereiche des Schaufelblattes als stützende Strukturen selektiv gehärtet oder andere Bereiche erweicht sind.

Entsprechende Strahlenquellen können ausreichend fokussiert werden, um die zu härtenden Bereiche selektiv und abgrenzbar entsprechend zu behandeln.

Vorteilhaft sind die stützenden Strukturen / ausgewählten Bereiche als Streben ausgebildet, die innerhalb des Schaufelblattes von einem rotationsachsnahen Punkt zu einem rotationsachsfernen Punkt verlaufen.

Die Streben können direkt in radialer Richtung oder auch in einem Winkel gegen die Längsachse des Rotors geneigt verlaufen. Sie können sich von der Nabe bis zum äußeren Ende des Schaufelblattes oder über eine Teilstrecke dieses Abstandes erstrecken. Auch ein mäanderförmiger oder Zick-Zack-Verlauf der Streben ist möglich, der durch Ausbildung geeigneter Biegezonen die Komprimierung des Rotors unterstützt.

Im Gehäuse können derartige als Streben ausgebildete Bereiche beispielsweise in Umfangsrichtung, in Längsrichtung parallel zur Rotationsachse oder auch helixförmig um die Rotationsachse umlaufend ausgebildet sein.

Verschiedene Streben können parallel zueinander, jedoch auch strahlenförmig auseinanderlaufend gestaltet sein. Mit den Streben können die Kraftlinien der in einem Schaufelblatt unter Belastung wirkenden Kräfte nachgebildet werden. In diesem Fall können mehrere Streben beispielsweise von einem Punkt oder einem Bereich aus strahlenförmig auseinanderlaufen.

Es kann auch vorteilhaft vorgesehen sein, dass zwischen den gehärteten Stützstrukturen und den nicht gehärteten Bereichen des Schaufelblattes oder des Gehäuses Übergangsbereiche vorgesehen sind, die einen geringeren Härtungsgrad aufweisen als die gehärteten Strukturen und einen höheren Härtungsgrad als die nicht gehärteten Bereiche.

Die Härtung der Stützstrukturen im Nachhinein nach Herstellung des Schaufelblattkörpers oder des Gehäuses gibt die Möglichkeit, einen allmählichen Materialübergang zwischen einem nachgiebigen und einem gehärteten Material innerhalb des Schaufelblattkörpers zu erzeugen.

Die gehärteten Bereiche können beispielsweise an der Oberfläche und auch nur ausschließlich an der Oberfläche des Schaufelblattes oder Gehäuses angeordnet sein, oder sie können in der Tiefe im Inneren des Schaufelblattes oder Gehäuses liegen.

Es können innerhalb der Schaufelblätter oder des Gehäuses auch ganze Teilflächen als stützende Strukturen gehärtet werden, wie beispielsweise kreisrunde oder ovale Scheiben, die im Belastungsfall beispielsweise eine äußerst stabile Kalottenform annehmen können.

Die Erfindung bezieht sich außer auf eine Blutpumpe der oben genannten Art mit den angeführten vorteilhaften Weiterbildungen auch auf ein Verfahren zur Herstellung einer Blutpumpe, bei dem der Rotor, insbesondere ein Schaufelblatt und/oder das Gehäuse, bei der Formgebung mittels eines Spritz-, Spritzguss-oder Extrusionsverfahrens oder eines Auftragverfahrens inhomogen hergestellt wird und/oder nach der Formgebung durch Strahlung in ausgewählten Bereichen bezüglich mechanischer Materialeigenschaften modifiziert wird.

Im Folgenden wird die Erfindung anhand eines Ausführungsbeispiels in einer Zeichnung gezeigt und nachfolgend beschrieben. Dabei zeigt
- Fig. 1: schematisch in einer Übersicht eine Herzkatheterpumpe, die wenigstens teilweise in ein Ventrikel eingeschoben ist,
- Fig. 2: in einem Längsschnitt den Aufbau einer Pumpe mit einem Rotor und einem Gehäuse,
- Fig. 3: das Gehäuse einer Pumpe,
- Fig. 4: in dreidimensionaler Ansicht einen Rotor einer Pumpe mit einer Nabe,
- Fig. 5: in dreidimensionaler Ansicht einen nabenlosen Rotor,
- Fig. 6: einen Ausschnitt aus einem Längsschnitt durch ein Pumpengehäuse sowie
- Fig. 7: ein durch verschiedenartige Streben verstärktes Gehäuse einer Pumpe.

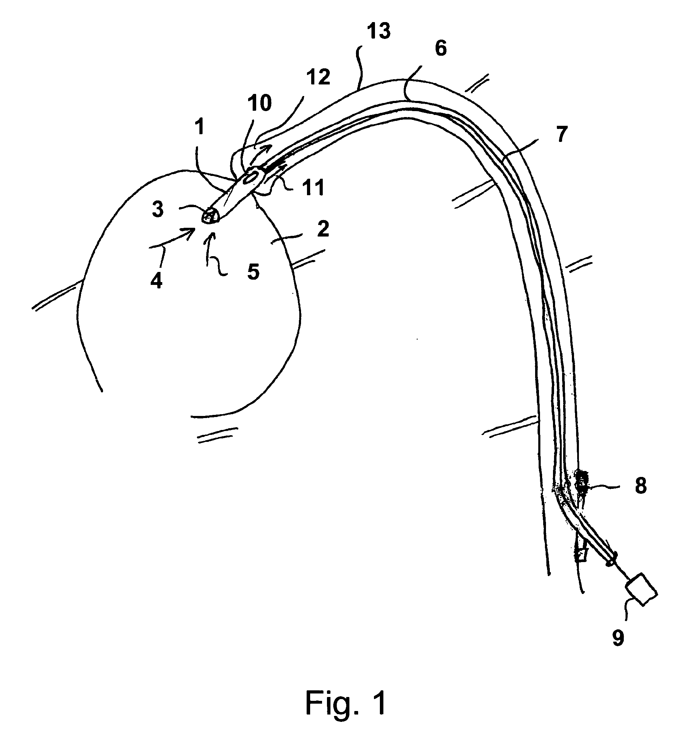

Fig. 1 zeigt eine Pumpe 1, die in ein Ventrikel 2 eines menschlichen Herzens, das lediglich schematisch dargestellt ist, hineinragt. Die Pumpe 1 weist einen Ansaugkäfig 3 auf, der Öffnungen zum Ansaugen von Blut freilässt. Das einströmende Blut ist durch die Pfeile 4, 5 symbolisiert.

Innerhalb der Pumpe 1 ist ein Rotor vorgesehen, der mit einer Geschwindigkeit zwischen 3000 und 50000 U/min rotiert und das Blut in Längsrichtung in das Blutgefäß 13 fördert. Dazu ist über die Pumpe ein Abströmschlauch geschoben, der Abströmöffnungen 10 proximal der Herzklappe aufweist, durch die das Blut in Richtung der Pfeile 11 abströmt. Die Pumpe 1 ist am Ende eines Hohlkatheters 6 gehalten, der durch eine Schleuse 8 in den Körper eines Patienten und dort direkt in ein Blutgefäß 13 eingeführt ist. Innerhalb des Hohlkatheters 6 ist eine Antriebswelle 7 geführt, die außerhalb des Körpers mit einem Motor 9 und in der Pumpe 1 mit einem Rotor verbunden ist.

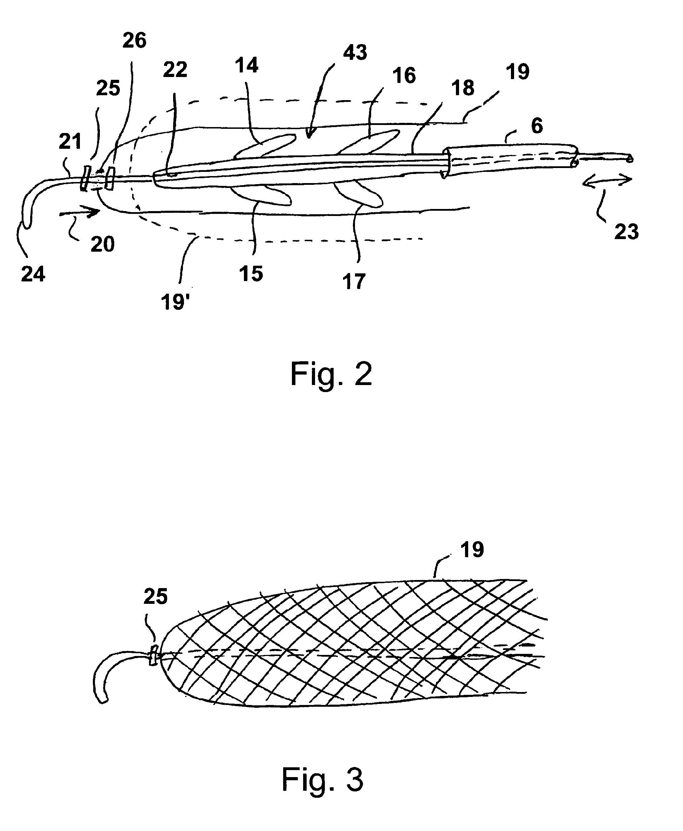

Fig. 2 zeigt in detaillierterer Darstellung den inneren Aufbau der Pumpe 1. Es ist ein Gehäuse 19 dargestellt, in dessen Innerem ein Rotor 43 angeordnet ist. Der Rotor 43 weist eine zentrale Nabe 18 auf, an der Schaufelblätter 14, 15, 16, 17 befestigt sind.

Um den Rotor radial komprimierbar und expandierbar zu machen, sind die Schaufelblätter 14, 15, 16, 17 radial an die Nabe 18 anlegbar, um den Radius der Pumpe zu verringern. In diesem Zustand ist auch das Gehäuse 19 in seinem Radius komprimiert.

Die Schaufelblätter sowie auch gegebenenfalls die Nabe 18 bestehen vorteilhaft aus einem elastischen nachgiebigen Werkstoff, der allerdings durch Streben oder Stützen verstärkt sein kann, so dass die Schaufelblätter einerseits wegen der Nachgiebigkeit eng an die Nabe angeklappt, andererseits aber bis zur vollen radialen Ausdehnung aufgerichtet werden können.

Üblicherweise werden die Schaufelblätter 14, 15, 16, 17 durch die Inbetriebnahme des Rotors bei Rotation infolge des auftretenden Fluidgegendrucks und/oder der Zentrifugalkräfte bis in den expandierten Zustand aufgerichtet. Alternativ dazu oder zusätzlich können auch Streben oder ein Gerüst aus einem Gedächtnismaterial wie beispielsweise Nitinol vorgesehen sein, die beim Übergang in die expandierte Gestalt die Schaufelblätter aufrichten. Während des Aufrichtens der Schaufelblätter kann durch diese auch das Gehäuse 19 in die gestrichelt in der Fig. 2 dargestellte Form 19' expandiert werden.

Um diesen Vorgang zu erleichtern und einen anderen Mechanismus zur radialen Expansion des Gehäuses 19 zu nutzen, ist gemäß der Erfindung ein Steuerkörper 21 vorgesehen, der in dem dargestellten Beispiel als Führungsdraht ausgebildet ist, eine Öffnung 22 zentrisch in der Nabe 18 durchsetzt und durch die Wand des Gehäuses 19 distal an dessen distalem Ende hindurchragt.

Der Steuerkörper ragt durch die Nabe 18 und den Hohlkatheter 6 hindurch und ist von außerhalb des Körpers betätigbar, d. h. in Längsrichtung verschiebbar. Der Steuerkörper weist einen ersten Anschlagkörper 25 distal von der Wand des Gehäuses 19 und einen zweiten Steuerkörper 26 proximal der Wand des Gehäuses 19 auf, wobei die Anschlagkörper 25, 26 eine zumindest teilweise größere radiale Ausdehnung haben als die durchsetzte Öffnung in dem Gehäuse 19.

Wird der Steuerkörper 21 in Richtung des Pfeils 20, also in die proximale Richtung, zurückgezogen, so wird damit das Gehäuse 19 in axialer Richtung unter Druck gesetzt und zusammengeschoben. Da das Gehäuse 19 aus einem beweglichen Geflecht, beispielsweise einem Drahtgeflecht oder einem Geflecht von Fasern mit teilweise helixartigem Verlauf, besteht, wird es bei einer derartigen axialen Kompression selbsttätig radial expandiert.

Damit ist das Gehäuse in die expandierte Form überführt, in der es für die expandierten Schaufelblätter genug Raum für die Rotation bietet.

Damit kann durch die Schaufelblätter 14, 15, 16, 17 entsprechend Fluid innerhalb des Gehäuses in Axialrichtung gefördert werden.

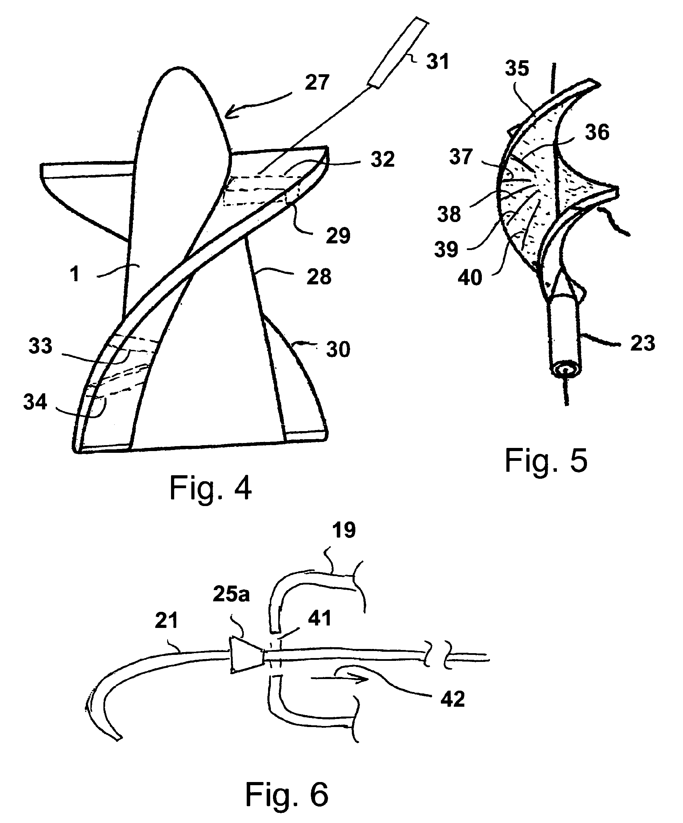

Der Anschlagkörper 25 kann derart gestaltet sein, dass er bei einem Zurückziehen in Richtung des Pfeils 42 in Fig. 6 zunächst eine Axialkompression des Gehäuses 19 bewirkt, sich bei darauf folgender größerer Kraftanwendung jedoch aufgrund der konischen Form in die Ausnehmung 41 des Gehäuses 19 hineinzieht und bei weiterer Steigerung der Axialkraft durch die Öffnung 41 hindurchgezogen werden kann. Dies kann dadurch unterstützt werden, dass der Körper 25a aus einem wenigstens teilweise nachgiebigen Material, beispielsweise einem Schaumstoff, besteht.

Die Anschlagkörper können auch beispielsweise aus einem Material bestehen, das sich im Laufe der Zeit in Körperflüssigkeiten, speziell Blut, auflöst, so dass der Steuerkörper, nachdem er seine Aufgabe der Kompression oder Expansion des Gehäuses 19 erfüllt hat, ohne Schwierigkeiten entfernt werden kann.

Der zweite Anschlagkörper 26 kann entsprechend dem Anschlagkörper 25 für eine axiale Expansion des Gehäuses 19 genützt werden, indem der Steuerkörper 21 entgegengesetzt dem Pfeil 20 in der Fig. 2 geschoben und damit auf das Gehäuse eine axiale Expansionskraft ausgeübt wird. Im Zuge dieser axialen Expansion durchläuft das Gehäuse 19 gleichzeitig eine radiale Kompression, im Zuge derer eine radiale Kompressionskraft auch auf die Schaufelblätter 14, 15, 16, 17 ausgeübt wird, so dass diese zusätzlich komprimiert werden.

In dem dargestellten Beispiel kann der Rotor 18 relativ zu dem Steuerkörper 21 frei rotieren, so dass der Steuerkörper 21 relativ zum Rotor in Bezug auf die Rotation feststehend ausgebildet ist.

Der Steuerkörper 21 kann alternativ zu dem in den Figuren dargestellten Beispiel auch zur Rotationslagerung des Rotors 43 am distalen Ende des Gehäuses 19 dienen.

Dazu kann der Steuerkörper 21 mit dem Gehäuse 19 unverschieblich verbunden und in einem axial festen Lager am Gehäuse 19 gelagert sein. Es kann andererseits auch der Rotor, insbesondere die Nabe 18, gegenüber dem Steuerkörper 21 durch ein zwischen diesen angeordnetes Wälzlager oder Gleitlager gelagert sein.

Fig. 4 zeigt in einer dreidimensionalen Ansicht einen Rotor 27 einer komprimierbaren und expandierbaren Blutpumpe mit einer Nabe 28, die zwei Schaufelblätter 29, 30 trägt. Die Schaufelblätter 29, 30 laufen schraubenförmig um die Nabe 28 um. Schaufelblätter und Nabe können beispielsweise aus demselben, flexiblen, insbesondere elastischen Material bestehen.

Die Schaufelblätter können auch kürzer ausgebildet sein als in der Abbildung dargestellt, so dass eine Vielzahl von Schaufelblättern am Umfang der Nabe 28 verteilt ist.

Innerhalb des Schaufelblattes 29 sind stützende Strukturen 32, 33, 34 in Form von integrierten Streben dargestellt, die innerhalb des Volumens des Schaufelblattes 29 angeordnet sind. Diese sind gemäß der Erfindung dadurch gebildet, dass innerhalb des Werkstoffes oder eines Werkstoffes des Schaufelblattes 29 bestimmte Bereiche gezielt und selektiv gehärtet sind.

Hierzu besteht das Schaufelblatt 29 wenigstens teilweise aus einem mittels Strahlung, insbesondere Licht, UV-Licht, Laserstrahlung, Röntgenstrahlung oder Alpha-, Beta- oder Gammastrahlung, härtbaren Werkstoff, beispielsweise einem Elastomer mit härtbaren Anteilen. Es kann sich dabei beispielsweise um einen strahlenvernetzbaren Kautschuk handeln.

In der Fig. 4 ist beispielhaft ein steuerbarer Laser 31 dargestellt, mittels dessen die Bereiche der Stützstrukturen gezielt bestrahlbar und damit im gewünschten Maß härtbar sind. Dabei können die Konturen der zu härtenden Bereiche scharf gestaltet werden, es ist jedoch auch denkbar, allmähliche Übergänge zwischen gehärteten und nicht gehärteten Bereichen zu schaffen, in denen der Werkstoff nur teilweise gehärtet wird.

Die Streben können vorteilhaft in radialer Richtung des Rotors in expandiertem Zustand angeordnet werden. Die Gestaltung der Streben durch die Bestrahlungshärtung kann vorteilhaft im kraftfreien Zustand oder im expandierten Zustand erfolgen, um die gewünschte Form und Ausrichtung der stützenden Strukturen möglichst genau zu erreichen.

Die Fig. 5 zeigt einen nabenlosen Rotor mit einem Schaufelblatt 35, in das Stützstrukturen 36, 37, 38, 39, 40 in Form von strahlenförmig verlaufenden Streben durch selektive Verfestigung eingebracht worden sind.

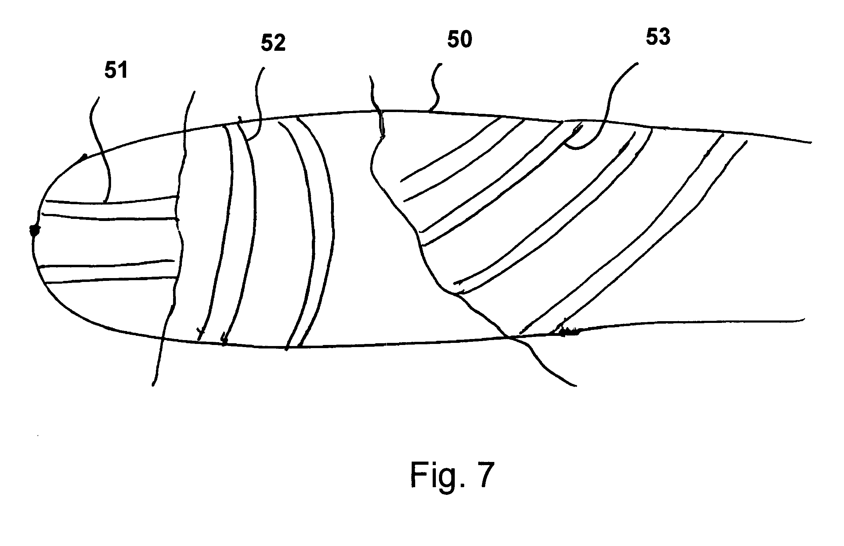

Fig. 7 zeigt ausgewählte, gehärtete Bereiche an einem Gehäuse 50 am Beispiel von parallel zur Rotationsachse verlaufenden Streben 51, in Umfangsrichtung verlaufenden Streben 52 und schraubenförmig umlaufenden Streben 53.

Die Gestaltung der Stützstrukturen mittels einer steuerbaren Bestrahlung hat gegenüber dem Einbringen von vorgefertigten festen Strukturen auch den Vorteil, dass sehr komplexe geometrische Gebilde als Stützstrukturen erzeugt werden können. Zudem entfällt ein Umspritzen von Streben mit einem Spritzgusswerkstoff, wobei die Streben durch Krafteinwirkung je nach der Viskosität des Spritzgusswerkstoffes verformt oder verschoben werden können.

Die Erfindung erlaubt durch Gestaltung von verschiedenen Elementen der Pumpe eine komfortable Kompression bzw. Expansion der Pumpe, verbunden mit einer optimierten Stabilität im expandierten Zustand und einer guten und reversiblen Komprimierbarkeit der Schaufelblätter.

Aspekte der Erfindung sind unter anderem:
1. Blutpumpe für die invasive Anwendung innerhalb eines Körpers eines Patienten mit einem um eine Rotationsachse antreibbaren radial komprimierbaren und expandierbaren Rotor (43, 27), der eine Nabe (18, 28) und wenigstens ein daran befestigtes Schaufelblatt (14, 15, 16, 17, 29, 30) aufweist, sowie mit einem Gehäuse (19, 19'), das durch eine Axialdehnung oder Axialkompression in radialer Richtung komprimierbar oder expandierbar ist, dadurch gekennzeichnet, dass ein die Nabe (18) in Längsrichtung durchsetzender Steuerkörper (21) vorgesehen ist, der auf der distalen Seite des Rotors mit dem Gehäuse derart gekoppelt ist, dass er durch eine Bewegung in Längsrichtung gegenüber dem Gehäuse auf dieses Zug- und/oder Druckkräfte ausübt.
2. Blutpumpe nach Aspekt 1, dadurch gekennzeichnet, dass der Steuerkörper (21) mit dem Rotor (18) rotiert.
3. Blutpumpe nach Aspekt 1, dadurch gekennzeichnet, dass der Steuerkörper (21) gegenüber dem Rotor (18) stillsteht.
4. Blutpumpe nach Aspekt 1 oder einem der folgenden, dadurch gekennzeichnet, dass der Steuerkörper (21) mit dem Gehäuse (19, 19¹) axial unverschiebbar verbunden ist.
5. Blutpumpe nach Aspekt 1, 2 oder 3, dadurch gekennzeichnet, dass der Steuerkörper (21) die Gehäusewand durchsetzt und auf deren distaler Seite einen Anschlagkörper (25) aufweist, der beim Zurückziehen des Steuerkörpers in proximaler Richtung eine Kompressionskraft in Axialrichtung auf das Gehäuse ausübt.
6. Blutpumpe nach einem der Aspekte 1, 2, 3 oder 5, dadurch gekennzeichnet, dass der Steuerkörper (21) auf der proximalen Seite der Gehäusewand einen Anschlagkörper (26) aufweist, der bei einer Schubbewegung in distaler Richtung eine axiale Expansionskraft auf die Gehäusewand ausübt.
7. Blutpumpe nach Aspekt 1 oder einem der folgenden, dadurch gekennzeichnet, dass der Steuerkörper (21) als Führungsdraht ausgebildet ist.
8. Blutpumpe nach Aspekt 1 oder einem der folgenden, dadurch gekennzeichnet, dass der distal oder proximal der Gehäusewand angeordnete Anschlagkörper (25, 26) auflösbar oder verformbar ist, derart, dass der Steuerkörper entfernbar ist.
9. Blutpumpe nach Aspekt 1 oder einem der folgenden, dadurch gekennzeichnet, dass das Gehäuse (19, 19¹) ein bewegliches Gittergeflecht aufweist.
10. Blutpumpe nach Aspekt 9, dadurch gekennzeichnet, dass das Gehäuse (19, 19') eine durch das Gittergeflecht gestützte Membran aufweist.
11. Blutpumpe für die invasive Anwendung innerhalb des Körpers eines Patienten mit einem Gehäuse sowie einem im Betrieb in diesem angeordneten antreibbaren Rotor (43, 27) mit wenigstens einem Schaufelblatt (14, 15, 16, 17, 29, 30, 35), wobei der Rotor und/oder das Gehäuse (19, 19¹) radial komprimierbar und expandierbar ist, dadurch gekennzeichnet, dass der Rotor und/oder das Gehäuse wenigstens teilweise aus einem Werkstoff besteht, der in ausgewählten Bereichen (32, 33, 34, 36, 37, 38, 39, 40) gegenüber anderen Bereichen derart modifiziert ist, dass er sich in den ausgewählten Bereichen bezüglich mechanischer Materialeigenschaften von den anderen Bereichen unterscheidet.
12. Blutpumpe nach Aspekt 11, dadurch gekennzeichnet, dass die ausgewählten Bereiche (32, 33, 34, 36, 37, 38, 39, 40) sich von den anderen Bereichen durch eine physikalische und/oder chemische Struktur unterscheiden.
13. Blutpumpe nach Aspekt 11 oder 12, dadurch gekennzeichnet, dass die ausgewählten Bereiche (32, 33, 34, 36, 37, 38, 39, 40) während des Formverfahrens modifiziert sind.
14. Blutpumpe nach Aspekt 11 oder 12, dadurch gekennzeichnet, dass die ausgewählten Bereiche (32, 33, 34, 36, 37, 38, 39, 40) nach dem Formverfahren modifiziert sind.
15. Blutpumpe nach Aspekt 11 oder einem der folgenden, dadurch gekennzeichnet, dass wenigstens ein Teil des Rotors (43, 27), insbesondere eines Schaufelblattes, und/oder wenigstens ein Teil des Gehäuses einen durch Strahlung erweichbaren oder erhärtbaren Werkstoff aufweist und dass durch selektive Strahlungshärtung ausgewählte Bereiche (32, 33, 34, 36, 37, 38, 39, 40) versteift oder durch selektive Strahlungserweichung entsprechend ausgewählte Bereiche erweicht sind.
16. Blutpumpe nach Aspekt 11 oder einem der folgenden, dadurch gekennzeichnet, dass der Werkstoff in den ausgewählten Bereichen durch Strahlung, insbesondere Alpha-, Beta- oder Gammastrahlung und/oder Wärmestrahlung, modifizierbar ist.
17. Blutpumpe nach Aspekt 11, 12 oder 13, dadurch gekennzeichnet, dass versteifte ausgewählte Bereiche (32, 33, 34, 36, 37, 38, 39, 40) Streben sind, die innerhalb des Schaufelblattes (29, 30, 35) von einem rotationsachsnahen Punkt zu einem rotationsachsfernen Punkt verlaufen.
18. Blutpumpe nach Aspekt 11 oder einem der folgenden, dadurch gekennzeichnet, dass zwischen den gehärteten Stützstrukturen (32, 33, 34, 36, 37, 38, 39, 40) und den nicht gehärteten Bereichen des Schaufelblattes (29, 30, 35) oder des Gehäuses Übergangsbereiche vorgesehen sind, die einen geringeren Härtungsgrad aufweisen als die gehärteten Strukturen und einen höheren Härtungsgrad als die nicht gehärteten Bereiche.
19. Blutpumpe nach Aspekt 11 oder einem der folgenden, dadurch gekennzeichnet, dass die ausgewählten Bereiche, insbesondere stützende Strukturen, an der Oberfläche des Schaufelblattes (29, 30, 35) oder des Gehäuses liegen.
20. Blutpumpe nach Aspekt 11 oder einem der folgenden, dadurch gekennzeichnet, dass die stützenden Strukturen im Inneren des Schaufelblattes (29, 30, 35) liegen.
21. Verfahren zur Herstellung einer Blutpumpe nach einem der Aspekte 11 bis 20, dadurch gekennzeichnet, dass der Rotor (43, 27), insbesondere ein Schaufelblatt (14, 15, 16, 17, 29, 30, 35) und/oder das Gehäuse (19, 19'), bei der Formgebung mittels eines Spritz-, Spritzguss- oder Extrusionsverfahrens oder eines Auftragverfahrens inhomogen hergestellt wird und/oder nach der Formgebung durch Strahlung in ausgewählten Bereichen (32, 33, 34, 36, 37, 38, 39, 40) bezüglich mechanischer Materialeigenschaften modifiziert wird.
22. Verfahren nach Aspekt 21, wobei das Spritzvefahren durch tropfenweises Auftragen des Werkstoffes bei Variation der Zusammensetzung der Tropfen gekennzeichnet ist.

## Patentansprüche

1. Blutpumpe für die invasive Anwendung innerhalb eines Körpers eines Patienten mit einem um eine Rotationsachse antreibbaren radial komprimierbaren und expandierbaren Rotor (43, 27), der eine Nabe (18, 28) und wenigstens ein daran befestigtes Schaufelblatt (14, 15, 16, 17, 29, 30) aufweist, sowie mit einem Gehäuse (19, 19'), das durch eine Axialdehnung oder Axialkompression in radialer Richtung komprimierbar oder expandierbar ist, **dadurch gekennzeichnet, dass** ein die Nabe (18) in Längsrichtung durchsetzender Steuerkörper (21) vorgesehen ist, der auf der distalen Seite des Rotors mit dem Gehäuse derart gekoppelt ist, dass er durch eine Bewegung in Längsrichtung gegenüber dem Gehäuse auf dieses Zug- und/oder Druckkräfte ausübt.

2. Blutpumpe nach Anspruch 1, **dadurch gekennzeichnet, dass** der Steuerkörper (21) mit dem Rotor (18) rotiert
oder dass der Steuerkörper (21) gegenüber dem Rotor (18) stillsteht
und/oder dass der Steuerkörper (21) mit dem Gehäuse (19, 19') axial unverschiebbar verbunden ist.

3. Blutpumpe nach Anspruch 1 oder 2, **dadurch gekennzeichnet, dass** der Steuerkörper (21) die Gehäusewand durchsetzt und auf deren distaler Seite einen Anschlagkörper (25) aufweist, der beim Zurückziehen des Steuerkörpers in proximaler Richtung eine Kompressionskraft in Axialrichtung auf das Gehäuse ausübt
und/oder dass der Steuerkörper (21) auf der proximalen Seite der Gehäusewand einen Anschlagkörper (26) aufweist, der bei einer Schubbewegung in distaler Richtung eine axiale Expansionskraft auf die Gehäusewand ausübt.

4. Blutpumpe nach Anspruch 1 oder einem der folgenden, **dadurch gekennzeichnet, dass** der Steuerkörper (21) als Führungsdraht ausgebildet ist.

5. Blutpumpe nach Anspruch 1 oder einem der folgenden, **dadurch gekennzeichnet, dass** der distal oder proximal der Gehäusewand angeordnete Anschlagkörper (25, 26) auflösbar oder verformbar ist, derart, dass der Steuerkörper entfernbar ist.

6. Blutpumpe nach Anspruch 1 oder einem der folgenden, **dadurch gekennzeichnet, dass** das Gehäuse (19, 19') ein bewegliches Gittergeflecht aufweist
und/oder dass das Gehäuse (19, 19') eine durch das Gittergeflecht gestützte Membran aufweist.

7. Blutpumpe für die invasive Anwendung innerhalb des Körpers eines Patienten mit einem Gehäuse sowie einem im Betrieb in diesem angeordneten antreibbaren Rotor (43, 27) mit wenigstens einem Schaufelblatt (14, 15, 16, 17, 29, 30, 35), wobei der Rotor und/oder das Gehäuse (19, 19') radial komprimierbar und expandierbar ist, **dadurch gekennzeichnet, dass** der Rotor und/oder das Gehäuse wenigstens teilweise aus einem Werkstoff besteht, der in ausgewählten Bereichen (32, 33, 34, 36, 37, 38, 39, 40) gegenüber anderen Bereichen derart modifiziert ist, dass er sich in den ausgewählten Bereichen bezüglich mechanischer Materialeigenschaften von den anderen Bereichen unterscheidet.

8. Blutpumpe nach Anspruch 7, **dadurch gekennzeichnet, dass** die ausgewählten Bereiche (32, 33, 34, 36, 37, 38, 39, 40) sich von den anderen Bereichen durch eine physikalische und/oder chemische Struktur unterscheiden.

9. Blutpumpe nach Anspruch 7 oder 8, **dadurch gekennzeichnet, dass** die ausgewählten Bereiche (32, 33, 34, 36, 37, 38, 39, 40) während des Formverfahrens modifiziert sind
oder dass die ausgewählten Bereiche (32, 33, 34, 36, -37, 38, 39, 40) nach dem Formverfahren modifiziert sind.

10. Blutpumpe nach Anspruch 7 oder einem der folgenden, **dadurch gekennzeichnet, dass** wenigstens ein Teil des Rotors (43, 27), insbesondere eines Schaufelblattes, und/oder wenigstens ein Teil des Gehäuses einen durch Strahlung erweichbaren oder erhärtbaren Werkstoff aufweist und dass durch selektive Strahlungshärtung ausgewählte Bereiche (32, 33, 34, 36, 37, 38, 39, 40) versteift oder durch selektive Strahlungserweichung entsprechend ausgewählte Bereiche erweicht sind
und/oder dass der Werkstoff in den ausgewählten Bereichen durch Strahlung, insbesondere Alpha-, Beta- oder Gammastrahlung und/oder Wärmestrahlung, modifizierbar ist.

11. Blutpumpe nach Anspruch 7, 8 oder 9, **dadurch gekennzeichnet, dass** versteifte ausgewählte Bereiche (32, 33, 34, 36, 37, 38, 39, 40) Streben sind, die innerhalb des Schaufelblattes (29, 30, 35) von einem rotationsachsnahen Punkt zu einem rotationsachsfernen Punkt verlaufen.

12. Blutpumpe nach Anspruch 7 oder einem der folgenden, **dadurch gekennzeichnet, dass** zwischen den gehärteten Stützstrukturen (32, 33, 34, 36, 37, 38, 39, 40) und den nicht gehärteten Bereichen des Schaufelblattes (29, 30, 35) oder des Gehäuses Übergangsbereiche vorgesehen sind, die einen geringeren Härtungsgrad aufweisen als die gehärteten Strukturen und einen höheren Härtungsgrad als die nicht gehärteten Bereiche.

13. Blutpumpe nach Anspruch 7 oder einem der folgenden, **dadurch gekennzeichnet, dass** die ausgewählten Bereiche, insbesondere stützende Strukturen, an der Oberfläche des Schaufelblattes (29, 30, 35) oder des Gehäuses liegen
und/oder dass die stützenden Strukturen im Inneren des Schaufelblattes (29, 30, 35) liegen.

14. Verfahren zur Herstellung einer Blutpumpe nach einem der Ansprüche 7 bis 13, **dadurch gekennzeichnet, dass** der Rotor (43, 27), insbesondere ein Schaufelblatt (14, 15, 16, 17, 29, 30, 35) und/oder das Gehäuse (19, 19'), bei der Formgebung mittels eines Spritz-, Spritzguss- oder Extrusionsverfahrens oder eines Auftragverfahrens inhomogen hergestellt wird und/oder nach der Formgebung durch Strahlung in ausgewählten Bereichen (32, 33, 34, 36, 37, 38, 39, 40) bezüglich mechanischer Materialeigenschaften modifiziert wird.

15. Verfahren nach Anspruch 14, wobei das Spritzvefahren durch tropfenweises Auftragen des Werkstoffes bei Variation der Zusammensetzung der Tropfen gekennzeichnet ist.
